(19) **Europäisches Patentamt / European Patent Office / Office européen des brevets**

(11) **EP 3 727 302 B1**

(12) **FASCICULE DE BREVET EUROPEEN**

(45) Date de publication et mention de la délivrance du brevet:
**20.04.2022 Bulletin 2022/16**

(21) Numéro de dépôt: **18833969.1**

(22) Date de dépôt: **18.12.2018**

(51) Classification Internationale des Brevets (IPC):
**A61K 8/34** *(2006.01)*  **A61Q 19/08** *(2006.01)*
**A61K 8/9794** *(2017.01)*

(52) Classification Coopérative des Brevets (CPC):
**A61Q 19/08; A61K 8/345; A61K 8/9794**

(86) Numéro de dépôt international:
**PCT/FR2018/053367**

(87) Numéro de publication internationale:
**WO 2019/122683 (27.06.2019 Gazette 2019/26)**

(54) **COMPOSITIONS COSMÉTIQUES NOTAMMENT A ACTIVITÉ ANTI-VIEILLISSEMENT COMPRENANT UN EXTRAIT VERT DE LA PLANTE AFRAMOMUM ANGUSTIFOLIUM OU LONGOZA.**

KOSMETISCHE ZUSAMMENSETZUNGEN, INSBESONDERE MIT ANTI-AGING-EFFEKT, MIT EINEM EXTRAKT AUS DER AFRAMOMUM-ANGUSTIFOLIUM- ODER LONGOZA-PFLANZE

COSMETIC COMPOSITIONS IN PARTICULAR WITH ANTI-AGING EFFECT, COMPRISING A GREEN EXTRACT OF AFRAMOMUM ANGUSTIFOLIUM OR LONGOZA PLANT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.12.2017 FR 1762974**

(43) Date de publication de la demande:
**28.10.2020 Bulletin 2020/44**

(73) Titulaire: **LVMH Recherche
45800 Saint Jean de Braye (FR)**

(72) Inventeurs:
• **NIZARD, Carine
94200 IVRY-SUR-SEINE (FR)**
• **JEANNETON, Olivier
45530 VITRY-AUX-LOGES (FR)**
• **LEBLANC-NOBLESSE, Emmanuelle
45800 SAINT JEAN DE BRAYE (FR)**
• **PECHER, Virginie
45800 SAINT JEAN DE BRAYE (FR)**

(74) Mandataire: **Cabinet Beau de Loménie
158, rue de l'Université
75340 Paris Cedex 07 (FR)**

(56) Documents cités:
**US-A1- 2010 055 217    US-B2- 7 381 436**

**Description**

La présente invention concerne essentiellement une composition cosmétique, notamment à activité anti-vieillissement, caractérisée en ce qu'elle comprend, à titre d'ingrédient actif, un extrait vert de graines de la plante *Aframomum angustifolium* ou *Longoza.*

L'invention concerne également l'utilisation d'un extrait vert de graines de la plante *Aframomum angustifolium* ou *Longoza,* comme agent cosmétique, notamment pour la fabrication d'une composition cosmétique, notamment ayant une activité anti-vieillissement.

Ci-après dans la description et les revendications, on utilisera de préférence par simplification le terme de *Longoza,* mais qui signifie évidemment indifféremment aussi la plante *Aframomum angustifolium.* En outre, on entend par extrait vert, un extrait avec un solvant procurant une compatibilité écologique et cosmétique de l'extrait, par exemple un solvant polaire comprenant ou constitué d'un polyol, en particulier un glycol, et plus particulièrement choisi parmi le propylène glycol, butylène glycol, pentylène glycol. Cet extrait est aussi vert, selon un mode de réalisation particulier, grâce à:

l'utilisation d'une ressource végétale renouvelable constituée par les graines de longoza ;
l'absence de déchets de solvants et notamment de solvants organiques type hexane ou heptane
l'utilisation de solvants alternatifs verts utilisés en cosmétiques comprenant ou constitué d'un polyol, en particulier un glycol, et plus particulièrement choisi parmi le propylène glycol, butylène glycol, pentylène glycol;

Un procédé d'extraction qui prévoit peu d'étapes avec une faible consommation d'énergie, en particulier en réalisant une première extraction à froid, une deuxième extraction à une température douce d'environ 55°C ; et

Le procédé devra éviter d'apporter un impact couleur, en gardant une faisabilité industrielle et sans augmenter le % de terpènes contenus dans le Longoza, pour éviter des risques toxicologiques.

## ETAT DE LA TECHNIQUE

On connaît par l'article de E.M. Gaydou et al. paru dans la Revue française des Corps Gras, N°1 de janvier 1983, pages 21 à 25, une étude sur la composition en acide gras des huiles extraites de graines provenant de quelques plantes de Madagascar, dont la plante *Aframomum angustifolium* (voir tableau 1, suite 2, page 24). Il s'agit d'un extrait au moyen d'un solvant apolaire des graines de la plante, en particulier à l'aide de l'hexane (voir page 24, "Partie expérimentale", 1er paragraphe).

On connaît aussi par le brevet ANDRE et al, LVMH RECHERCHE, US 7,381,436B2 une composition cosmétique contenant un extrait de graines de la plante *Aframomum angustifolium* ou LONGOZA avec un solvant polaire constitué en pratique par un mélange hydro-alcoolique éthanol/eau dans un rapport volumique 70/30. Ce document décrit une activité anti-vieillissement par une action de stimulation de la synthèse de collagène, en particulier de collagène VII présent au niveau de la jonction dermo-épidermique ou JDE, et/ou par une action sur les fibres oxytalanes et/ou les fibres élaunines ; et aussi de protéger la peau contre un vieillissement résultant de l'action des rayonnements actiniques, notamment dû aux rayons ultraviolets.

Egalement, le document US 2010/0055217 NIZARD, LVMH RECHERCHE est similaire au brevet ANDRE ci-dessus en décrivant un extrait hydro-alcoolique de LONGOZA mais utilisé avec le Lipochronae-6 pour obtenir u effet de synergie.

On connaît encore par l'article de Wiley et al dans Cell Metab. 2016, du 9 février ; 23(2) :303-314 le résultat de recherches prouvant que le dysfonctionnement des mitochondries augmente avec l'âge, ce qui induit leur sénescence avec un phénotype de sécrétion différent.

## BUTS DE L'INVENTION

L'invention a pour but de résoudre le problème technique consistant en la fourniture d'une solution permettant de trouver de nouveaux extraits ou principes actifs à partir de la plante *Aframomum angustifolium* ou LONGOZA, comme agent cosmétique ayant une bonne activité anti-vieillissement, ou une activité anti-vieillissement améliorée par rapport aux agents cosmétiques anti-vieillissement antérieurement connus incluant l'extrait hydro-alcoolique de la plante *Aframomum angustifolium* ou LONGOZA, et/ou ayant une très bonne compatibilité dans le cadre d'une application topique dans un but cosmétique, notamment sur la peau, et/ou n'ayant pas ou sensiblement pas d'effets secondaires ou irritants.

L'invention a aussi pour but de résoudre le problème technique énoncé ci-dessus à l'aide de nouveaux extraits ou principes actifs à partir de la plante *Aframomum angustifolium* ou LONGOZA capables de ralentir ou empêcher un dysfonctionnement des mitochondries conduisant à leur sénescence et à un vieillissement prématuré de la peau.

Encore, l'invention a pour but de résoudre les problèmes techniques énoncés ci-dessus en fournissant aussi un procédé d'obtention du principe actif compatible avec l'environnement en évitant l'emploi de solvant nocif à l'environnement, peu coûteux en énergie, et permettant de préparer des quantités suffisantes pour une utilisation à l'échelle industrielle et notamment à l'échelle cosmétique.

RESUME DE L'INVENTION

L'invention apporte une solution aux problèmes techniques énoncés ci-dessus, d'une manière simple, peu coûteuse utilisable à l'échelle industrielle et notamment à l'échelle cosmétique.

Selon un premier aspect, la présente invention fournit une composition cosmétique, caractérisée en ce qu'elle comprend, à titre d'ingrédient actif, un extrait, dit extrait vert, de graines de la plante *Aframomum angustifolium* ou *Longoza* réalisé avec un solvant polaire écologiquement compatible, et cosmétiquement acceptable, comprenant ou constitué d'un solvant polaire d'un polyol, en particulier un glycol, dans un excipient cosmétiquement acceptable.

Selon un second aspect, l'invention concerne également l'utilisation d'un extrait, dit extrait vert, de graines de la plante *Aframomum angustifolium* ou *Longoza* réalisé avec un solvant compatible avec l'environnement et cosmétiquement acceptable, comprenant ou constitué d'un solvant polaire d'un polyol, en particulier un glycol, comme agent cosmétique, pour la fabrication d'une composition cosmétique.

Selon un mode de réalisation particulier de l'invention, l'extrait vert de *Longoza* est utilisé comme agent cosmétique ayant une activité anti-vieillissement.

Selon une variante de réalisation avantageuse de l'invention, on utilise l'extrait vert de graines de la plante *Longoza,* pour une activité anti-rides, notamment pour diminuer, éliminer, ou ralentir l'apparition de rides, une action préservant ou restaurant la structure de la peau, notamment par une action de stimulation de la synthèse de ATP synthase 6 par les mitochondries.

Ainsi, l'invention permet d'avoir un effet de protection, de réparation ou de restauration de l'activité des mitochondries évitant leur sénescence résultant en un effet anti-vieillisssement, en particulier anti-rides.

L'invention permet aussi de protéger la peau contre un vieillissement résultant de l'action des rayonnements actiniques, notamment dû aux rayons ultraviolets.

Selon un troisième aspect, l'invention concerne un nouveau procédé d'extraction des graines de *Aframomium angustifolium* ou Longoza.

Dans le cadre de l'invention, on réalise l'extraction des graines de la plante *Aframomum angustifolium* ou *Longoza* avec un solvant polaire écologiquement compatible, et cosmétiquement acceptable, en particulier un solvant polaire comprenant ou constitué d'un polyol, en particulier un glycol, en obtenant ainsi un extrait « vert ».

Pour chacun des aspects de l'invention, le polyol utilisé peut présenter la formule chimique générale HO-R-OH, dans laquelle R peut représenter un radical hydrocarboné -(CH2)n avec n ayant de 2 à 16 atomes de carbone, en particulier de 3 à 8 atomes de carbone.

Selon un mode de réalisation particulier de l'invention, on utilisera comme polyol, un glycol courant largement disponible dans l'industrie et bon marché comme par exemple le propylène glycol, le butylène glycol ou 1, 3-butylène glycol, le pentylène glycol, et leur(s) mélange(s).

Selon une autre variante de réalisation, le broyage des graines est réalisé jusqu'à obtenir une poudre fine. Les grains de cette poudre présentent avantageusement un diamètre compris entre 0,01 et 1 mm environ.

Selon encore une autre variante de réalisation, on peut procéder à plusieurs extractions successives jusqu'à épuisement de la matière à extraire par le solvant polyol, en particulier un glycol.

Selon une variante de réalisation, l'extraction avec le polyol, en particulier un glycol a lieu une première fois à la température ambiante dite « à froid» et une deuxième fois à une température d'environ 55°C.

Selon un mode de réalisation particulier de l'invention, le procédé d'extraction comprend au moins une étape d'extraction des graines sèches broyées avec un solvant polaire constitué à 100% en polyol, en particulier un glycol. Selon une variante de réalisation, le rapport plante/ solvant m/v peut être de 1/1.75.

Selon une autre variante de réalisation, avant l'extraction proprement dite, on réalise une imprégnation des graines broyées par macération douce de par exemple 12 à 15 Heures à température ambiante dans le polyol, en particulier un glycol, en permettant ainsi de rendre plus accessibles et plus disponibles les molécules d'intérêt.

Selon encore une variante, on réalise, après la macération, une première filtration pour obtenir un premier filtrat et un gâteau des graines broyées, puis on réalise sur le gâteau une extraction à température douce, par exemple de l'ordre de 55°C pendant une durée de plusieurs heures, par exemple environ 3 heures puis une filtration est faite pour obtenir un deuxième filtrat, par exemple sur un filtre de porosité 20-25 micromètres. Selon une variante particulière, les deux filtrats obtenus sont rassemblés et peuvent être à nouveau filtrés sur un filtre stérilisant ou non, par exemple de 0.5 à 0.1 micromètres. On peut ajuster la solution obtenue avec du polyol, en particulier un glycol, ou autre solvant biocompatible et cosmétiquement acceptable pour aboutir à environ 1% en poids d'extrait sec.

Selon un mode particulier de réalisation de ce procédé d'extraction, l'extrait au polyol, en particulier un glycol, peut constituer en lui-même l'ingrédient ou l'agent actif selon l'invention.

On peut, selon une autre variante particulière, évaporer sensiblement complètement le solvant d'extraction en obtenant un extrait sec que l'on peut soit utiliser tel quel comme l'ingrédient actif précité, soit le dissoudre à nouveau dans un solvant compatible avec l'environnement et cosmétiquement acceptable, tel qu'un un polyol, en particulier un glycol, ou un alcool ou un mélange hydro-alcoolique afin d'obtenir une autre variante de l'ingrédient actif précité.

Selon une autre variante avantageuse de l'invention, le solvant utilisé pour la redissolution est un glycol.

Selon un mode de réalisation particulier de l'invention, comme indiqué précédemment, on utilisera un glycol courant largement disponible dans l'industrie et bon marché comme par exemple le propylène glycol, le butylène glycol ou 1, 3-butylène glycol, le pentylène glycol, et leur(s) mélanges. Une variante de réalisation particulière utilise le butylène glycol.

Dans le cadre de l'invention, l'extrait obtenu est facile à utiliser comme agent cosmétique et à mélanger avec les autres ingrédients de composition cosmétique à préparer, que ce soit pour incorporer dans une phase aqueuse ou une phase grasse.

Selon un cinquième aspect, la présente invention concerne aussi un procédé de soin cosmétique, caractérisé en ce qu'il comprend l'application topique sur la peau d'une personne en ayant besoin, d'une quantité cosmétiquement efficace d'un extrait, dit extrait vert, de graines de la plante *Aframomum angustifolium* ou *Longoza,* tel(les) que précédemment défini(es).

Selon un mode de réalisation avantageux de l'invention, ce procédé de soin cosmétique est mis en œuvre pour appliquer l'extrait de graines de la plante *Aframomum angustifolium* ou *Longoza* précité sur les zones de la peau, ayant besoin d'un soin anti-vieillissement, en particulier pour lutter contre les effets du vieillissement résultant d'un dysfonctionnement des mitochondries.

Il a été découvert de manière inattendue que l'extrait vert de l'invention au glycol a une action significative sur le métabolisme énergétique. L' Adénosine TriPhosphate ou ATP synthase est un complexe enzymatique qui se trouve dans les crêtes mitochondriales. Le rôle de cette enzyme est de synthétiser l'ATP à partir du gradient électrochimique et de l'Adénosine DiPhosphate ou ADP qui est entretenu par la chaine respiratoire ainsi que du Phosphate Inorganique (« PI »), pour obtenir ADP + PI = ATP.

L'ADP et le PI sont des véritables moteurs moléculaires indispensables à la vie des cellules. L'ATP est la seule forme d'énergie chimique convertie en énergie chimique et mécanique.

L'invention a ainsi de manière inattendue une action préservant ou restaurant la structure de la peau, un effet de protection, de réparation ou de restauration de l'élasticité et/ou de la fermeté de la peau, notamment par une action de stimulation de la sous-unité synthase 6 de l'ATP.

L'invention a aussi une activité anti-vieillissement qui vise plus particulièrement une activité anti-rides, pour diminuer, éliminer, ou ralentir l'apparition de rides.

Dans le cadre de l'un quelconque des aspects de l'invention, ledit extrait vert est présent à une concentration, exprimée en extrait sec ou en substance, comprise entre 0,001 % et 5 %, en particulier entre 0,01 % et 1%, en poids par rapport au poids total de la composition.

D'autres buts, caractéristiques et avantages de l'invention apparaîtront clairement à l'homme de l'art à partir de la description suivante contenant plusieurs modes de réalisation de l'invention donnés à titre d'exemple et qui ne sauraient en aucune façon limiter la portée de l'invention.

Dans les exemples ci-après, tous les pourcentages sont donnés en poids, la température est la température ambiante, soit 25°C, ou est donnée en degré Celsius, la pression est la pression atmosphérique, sauf indication contraire.

**Exemple 1 Selon l'invention**

Exemple de préparation **en laboratoire** d'un extrait vert de graines de la plante *Aframomum angustifolium* ou *Longoza,* avec un glycol par exemple le butylène glycol, selon l'invention,

A partir de 200 g de graines de la plante *Longoza* de différents lots du commerce, on réalise la procédure d'extraction suivante :

a) tout d'abord, on effectue un broyage des graines séchées du commerce jusqu'à par exemple une dimension moyenne de poudre comprise entre 0.01 mm et 1 mm.

b) ensuite, on procède à une macération une nuit soit environ 12Heures, dans 350 mL de Butylène glycol, soit un rapport graines/solvant (m/v) de 1/1.75 à température ambiante et statique.

c) on réalise, après la macération, une première filtration par exemple sur un filtre de porosité 20-25 micromètres, pour obtenir un premier filtrat en obtenant une solution opaque orange-rouge avec un résidu sec autour de 20g/L ou jus de macération, et un gâteau des graines broyées, puis

d) on réalise sur le gâteau avec encore 350mL de butylène glycol, une extraction à température douce, par exemple de l'ordre de 55°C pendant une durée de plusieurs heures, par exemple environ 3 heures, puis

e) une deuxième filtration est faite par exemple sur un filtre de porosité 20-25 micromètres, pour obtenir un deuxième filtrat, en obtenant une solution orange opaque avec un résidu sec autour de 5g/L .

f) Selon une variante particulière, les deux filtrats ou extraits obtenus sont rassemblés et peuvent être à nouveau filtrés sur un filtre stérilisant, par exemple de 0.5 à 0.1 micromètres. Le filtrat est une solution orange orangée limpide sur lequel on peut procéder aux analyses de dosage en principe actif et une chromatographie sur couche mince et une microbiologie.

g) On peut ajuster la solution de filtrat ou extrait obtenu(e) avec du butylène glycol ou autre solvant biocompatible et cosmétiquement acceptable pour aboutir à un taux de matières sèches de 10g/L soit environ 1% en poids d'extrait sec.

Cet extrait butylène glycol constitue en lui-même selon un mode de réalisation particulier, l'extrait vert de graines de *Longoza* ou de *Aframomum angustifolium* selon l'invention utilisable comme agent cosmétique ou à titre d'ingrédient actif pour la fabrication d'une composition cosmétique comme montré dans les exemples suivants (extrait 1g). Cette solution butylène glycol contient 1 % en poids d'extrait sec.

h) pour l'incorporation de cet extrait sec dans une composition cosmétique selon l'invention, il est aussi possible d'en préparer une solution extemporanée au moment de fabriquer ladite composition, dans un solvant cosmétiquement acceptable, notamment dans de l'eau, de l'alcool, du butylèneglycol 1-3 ou dans un mélange de ces solvants. Ce mode de réalisation permet ainsi de préparer des solutions d'extrait vert sec de la plante *Aframomum angustifolium* ou *Longoza à* la concentration voulue.

i) on peut enfin procéder à une opération de conditionnement des différentes solutions de l'extrait décrites ci-dessus, dans l'attente de leur utilisation pour la fabrication des compositions cosmétiques recherchées. Dans ce cadre, il est recommandé de réaliser préalablement une filtration stérilisante sur filtre ayant un diamètre de pores de 0,22 $\mu$m avant le conditionnement dans des flacons de volumes appropriés, puis le stockage dans une chambre froide, par exemple à 4° C.

En moyenne, à partir de 1Kg de graines broyées de *Longoza* ou de *Aframomum angustifolium,* on obtient environ 3 à 3,5Kg d'extrait à 1% en poids de matières sèches dans du butylène glycol, ce qui correspond à 30 à 35grammes d'extrait sec. Le rendement d'extraction massique obtenue est en moyenne de 35% calculé comme suit :

$$RDT\ massique = masse\ d'extrait\ sec\ contenu\ dans\ l'extrait\ final\ *100/\ masse\ de\ graines\ broyées\ introduite\ au\ départ.$$

**Exemple 2 selon l'invention**

Exemple de préparation **sur pilote industriel** d'un extrait vert de graines de la plante *Aframomum angustifolium* ou *Longoza,* avec un glycol, par exemple le butylène glycol, selon l'invention

**1) Pilotes**

Les pilotes sont prévus pour traiter 10kg par pilote des graines d'Aframomum angustifolium en provenance de MADAGASCAR selon 3 lots de graines identifiés G1, G2 et G3 pour les Pilotes 1, 2 et 3, et des systèmes de filtrations adéquats décrits ci-après.

**2) Procédure d'extraction selon l'invention dans les pilotes**

L'extrait selon l'invention est réalisé avec un solvant polaire constitué de 100% butylène glycol à partir de graines sèches d'*Aframomum angustifolium* préalablement broyées traditionnellement avec un ratio plante/solvant (m/v) de 1/1.75 en faisant tout d'abord une imprégnation du végétal par macération douce de 12 à 15H à température ambiante dans le butylène glycol pour rendre plus accessible et disponibles les molécules d'intérêt. Une première étape de filtration est réalisée pour obtenir un filtrat 1.

Une deuxième extraction est réalisée à 55°C sur le gâteau pendant 3H puis une filtration est faite sur filtre de porosité de 20-25 $\mu$m pour obtenir un filtrat 2.

Les deux filtrats 1 et 2 obtenus sont rassemblés et à nouveau filtrés sur filtre de 0.5 à0.1$\mu$m.

La solution obtenue est ajustée avec du butylène glycol pour être à 1% d'extrait sec.

On obtient une solution orangée limpide glycolique.

les 3 extraits des 3 lots G1, G2 et G3, obtenus avec les 3 pilotes, sont dénommés E1, E2 et E3.

En moyenne, à partir de 10 Kg de graines broyées, il est obtenu environ 30 à 35Kg d'extrait à 1% de MS dans du butylène glycol (ce qui correspond à 300 à 350 g d'extrait sec).

Le rendement d'extraction massique obtenue est en moyenne de 35%. Il est calculé de cette façon :

RDT massique = masse d'extrait sec contenu dans l'extrait final *100/ masse de graines broyées introduit au départ.

### 3) Analyses physicochimiques des extraits

3-1) Masse Sèche (MS) comparable : 0.96 à 1% ( 9.6g/L à 10.0g/L)
pH de l'ordre de 6.6; Couleur comparable : 7.8 à 7.9 (Gardner)
Valeurs Lab 87.8-88.9 ; -3.3 à -2.5;67.7 à 69.3.

3-2)Polyphénols totaux :Méthode de la Pharmacopée européenne 2.8.14 La prise d'essai de 0.05g par échantillon. La lecture est faite à 750nm dans le visible.

| | Concentration g/ L en équivalent d'acide gallique |
|---|---|
| E1 | 0,012 |
| E2 | 0,017 |
| E3 | 0,016 |

On obtient des teneurs entre 0.012 et 0.016g/L. On observe que les teneurs sont légèrement plus importantes pour les extraits mis en oeuvre avec les extraits des graines E2 et E3.

### 4) Dosages des sucres totaux

Méthode de dosage à l'Anthrone sulfurique.La lecture est faite à 620nm .

| | Teneurs en % en équivalent de glucose |
|---|---|
| E1 | 5.9 |
| E2 | 7.0 |
| E3 | 8.1 |

Les teneurs sont légèrement plus importantes pour les extraits mis en œuvre avec les extraits des graines E2 et E3.

**Exemple 3 comparatif** entre le procédé selon l'invention de l'exemple 2 obtenant un extrait vert au butylène glycol de graines de la plante *Aframomum angustifolium* ou *Longoza* , selon l'invention et le procédé selon l'exemple 1 de US 7,381,436 obtenant un extrait à l'alcool/eau 70/30 en volume, de graines de la plante *Aframomum angustifolium* ou *Longoza*

**3-1) Pour réaliser cet essai comparatif, il a été utilisé les 2 échantillons suivants** :

a) un lot comparatif de graines de la plante de Madagascar *Aframomum angustifolium* ou *Longoza* dénommé_lot SX000HH qui est soumis au procédé d'extraction décrit à l'exemple 1 de US 7,381,436 qui utilise comme solvant d'extraction un mélange hydro-alcoolique ethanol/eau 70/30 en volume et l'extrait est dénommé : extrait lot SX000HH
b) l'extrait vert E2 selon l'exemple 2 de l'invention ci-dessus

**3-2) Détermination de l'activité anti-vieillissement** de l'extrait vert E2 de l'Exemple 2 de *Longoza* (*Aframomum angustifolium*) sur des cellules de Kératinocytes Humains Normaux, en abrégé KHN par action sur la synthèse de sous-unité synthase 6 de l'ATP par les mitochondries.

1. Introduction

Une étude sur les effets transcriptionnels de l'extrait vert butylène glycol de Longoza (Aframomum angustifolium) de l'exemple 1 comparé à l'extrait antérieur hydro-alcoolique éthanol/eau 70/30 en volume selon l'exemple 1 du brevet US 7,381,436B2, ont été évalués à l'aide de la technologie TLDA (TaqMan Low Density Array). Grâce à cette technologie, la modulation de l'expression de 96 gènes codant pour des protéines spécifiques est étudiée en réponse à un traitement de Kératinocytes Humains Normaux.

Après 24 heures de traitement, les tapis cellulaires ont été lysés afin de récupérer les ARNs. Ces ARNs ont été quantifiés et normalisés pour réaliser la Reverse Transcriptase. Les ADNc obtenus ont ensuite été préparés pour être analysés sur carte microfluidique TLDA FHN, KHN et MHN, 96 gènes «profil chronobiologie ».

2. Matériels & Méthode

2.1. Préparation des actifs testés et des contrôles les actifs testés

Actif 1 selon l'invention : extrait VERT E2 à 100% de Butylène glycol de graines de Longoza de madagascar selon l'exemple 2 ci-dessus.

Actif 2 comparatif : extrait au mélange EtOH/H2O 70/30 v/v de graines de Longoza de Madagascar selon l'exemple 1 de US 7,381,436B2 et l'exemple 3-1 a) ci-dessus.

Les concentrations finales testées sont de 0.0125% en poids pour l'actif 1 et l'actif 2

2.2. Traitements des cellules

L'étude a été effectuée sur des kératinocytes humains normaux issus d'une plastie abdominale d'un donneur féminin de 31 ans (code 1477). Les cellules ont d'abord été ensemencées dans du milieu Epilife complet (supplément en HKGS) à passage 4 (P4), à une densité de 125 000 cellules par puits, dans des plaques 12 puits. Les cellules sont ensuite traitées par la Perle de Madagascar Green 0.0125% et la Perle de Madagascar 0.0125% ainsi que leur témoin excipient respectif (BG 100% et EtOH/H2O 70/30 v/v).

Après 4 heures de traitement, les cellules sont récupérées afin d'en extraire les ARN totaux.

2.3. Technique TLDA (TaqMan Low Density Array)

2.3.1. Obtention des ARN totaux à l'aide du MicrolabSTAR (Hamilton)

Le milieu de culture des cellules est éliminé, et 250 μL de tampon de lyse RLT (fourni dans le kit Nucleospin RNA trace, (Réf.740709, lot n°1412/001, Macherey-Nagel) sont ajoutés. Les cellules sont raclées à l'aide d'un Cell Scraper puis le lysat cellulaire, récupéré dans une deepwell 1,2 mL (fourni dans le kit Nucleospin RNA). Les ARN totaux sont extraits selon les protocoles mis au point préalablement (NS_96RNA_KHN ou NS_96RNA_FHN.dws).

Les solutions d'ARN totaux obtenues sont dosées, et leur qualité vérifiée, à l'aide d'un lecteur de microplaques, le spectrostarNANO (BMG Labtech) couplé au MicrolabSTAR. Cet appareil est relié à l'ordinateur pilotant la plateforme robotique et possède un logiciel spécifique d'analyse des résultats (logiciel MARS). La technique nécessite une micro-plaque de 384 puits (384 microclear plate black LoBase ref : 788876 lot : 1305055 Greiner), un contrôle positif (RNA 250 ref : AM7155 lot 1301028 (FHN), lot : 1208025 (KHN), lot : 1208026 (MHN), Thermofisher) permettant de valider les pipetages réalisés par le robot ainsi que les valeurs générées par le lecteur spectrostarNANO.

2.3.2. Synthèse des ADN complémentaires

Le kit de Transcription inverse ou en anglais « Reverse Transcription » (RT) utilisé est le Kit de la marque « High Capacity Reverse Transcription Kit » (Réf. 4368813, lot n°00288647 ; Thermo Fisher) et a été employé selon le protocole fourni : 500 ng d'ARN totaux sont dilués dans de l'eau pour un volume final de 25 μL Ils sont ensuite incubés pendant 10 minutes à 25°C puis 2 heures à 37°C en présence de 25 μL de mélange réactionnel de High Capacity Reverse Transcription Kit 2X préalablement préparé comme indiqué ci-dessous. Les différentes incubations sont faites au sein du TRobot (Biométra).

Tableau 2: Préparation du mélange réactionnel High Capacity Reverse Transcription Kit 2X pour 1 réaction

| Réactifs | Tampon RT | dNTP | Sonde dite Random Primer | Enzyme ditRNase out | RT | H2O |
|---|---|---|---|---|---|---|
| Volume | 5 μL | 2 μL | 5 μL | 0.5 μL | 2.5 μL | 10 μL |

### 2.3.3. PCR-TaqMan Low Density Array

15 μL de chaque RT sont mélangés à 60 μl d'eau puis 75 μL de TaqMan Gene Expression master mix (Ref. 4369016, lot n°1507235, Thermo Fisher) sont ajoutés. Après homogénéisation, 100 μL sont déposés sur les cartes microfluidiques profil chronobiologie 96 gènes (lot B1885 (FHN), lot B1884 (KHN) et lot B1942 (MHN)), ces dernières sont centrifugées puis scellées.
Le CD, correspondant au profil des gènes déposés sur les plaques, est chargé dans le logiciel SDS 2.4 ce qui permet donc de préciser l'emplacement de chaque gène sur la carte. Les gènes contrôles (appelé « endogeneous » gene dans le logiciel) à utiliser pour la normalisation des résultats sont indiqués avant le lancement de la PCR. Cette dernière est réalisée selon le protocole fourni par Applied Biosystems dans l'appareil ABI Prism 7900HT Sequence detection system. Les étapes de la qPCR sont 2 min à 50°C, 10 min à 94,5°C puis 30s à 97°C et 1 min à 59,7°C pour 40 cycles.

### 2.3.4. Analyse statistique des résultats

La PCR quantitative en temps réel peut être exploitée si son efficacité est comprise entre 90% et 110%.
Dans la méthode de RT-PCR -TLDA, la quantification est effectuée en utilisant la méthode comparative des ΔCt, soit la quantification relative (RQ). Cette méthode détermine le Ct de chaque gène de la carte en utilisant le logiciel RQ Manager qui prend en compte le bruit de fond pour chaque gène. Ce Ct est normalisé par rapport au Ct d'un gène de ménage (dans notre étude Beta-2-microglobuline) qui est étudié en parallèle sur la carte. Ce gène étant invariant dans les cellules, ses variations traduisent les variabilités de l'expérience (dosage des ARNs totaux, pipetages, étape de réverse transcription, dépôts dans la carte microfluidique, qualité de la carte, PCR dans l'appareillage). Avec ce logiciel RQ Manager, nous obtenons les valeurs de RQ (Relative Quantification) qui correspondent au niveau d'amplitude (x fois plus ou moins que le contrôle) de l'expression par rapport à notre contrôle. Ce dernier peut être le témoin non traité ou l'excipient. Le RQ est obtenu par le calcul suivant (la valeur du non traité étant égale à 1) :

$$RQ = 2^{-\Delta\Delta Ct} = 2^{-(\Delta Ct\ traité\ -\ \Delta Ct\ non\ traité)}$$

$$\Delta Ct\ traité = Ct\ gène\ cible\ traité - Ct\ gène\ de\ ménage\ traité$$

ΔCt non traité Ct gène cible non traité - Ct gène de ménage non traité
Pour que cette méthode soit fiable, il est impératif que l'efficacité de l'amplification du gène d'intérêt et du gène de ménage soit la même, ceci a été vérifié lors des mises au point effectuées avant l'utilisation des plaques en routine.
Afin d'évaluer des variations d'activité transcriptionnelle statistiquement significatives, nous utiliserons le test t de Student. Chaque condition est réalisée en triplicate (3 non traitées et 3 traitées dans les mêmes conditions). Le test-F de Fischer est tout d'abord appliqué en comparant les deux matrices de données. Lorsque la valeur est supérieure à $\alpha = 0,05$ alors la variance du test t de Student est de 2, lorsque le test-F de Fischer est inférieur à $\alpha = 0,05$, alors la variance sera égale à 3. Les variations transcriptionnelles retenues seront celles qui correspondent à un test t de Student inférieur à $\alpha = 0,05$.

### Tableau 3: Tableau codification couleur

- RQ<0.5 0.5>RQ<0.750 0.75>RQ<1 1>RQ<1.5 1,5>RQ<2 RQ>2

Un critère prenant en compte l'effet attendu (+ pour une augmentation,-pour une inhibition) permet de réaliser une première interprétation de résultats

- si l'effet obtenu va dans le sens attendu alors le code de dégradé de gris est appliqué comme ci-dessus
- si le résultat obtenu est inverse à l'effet attendu, le code de dégradé de gris n'est pas appliqué mais le résultat reste visible puisqu'il s'agit d'un résultat significatif

L'ensemble de ces données nous permet de noter les extraits les uns par rapport aux autres puisque la macro Excel indique le nombre de gènes modulés dans le sens attendu / nombre de gènes modulés au total. Dans le cadre d'un screening, cette note peut servir de critère de sélection.

Résultats relatifs à l'Effet de l'extrait E2 Longoza vert au butylène glycol selon l'invention de l'exemple 2, comparé au Longoza antérieur au mélange hydra-alcoolique Ethanol/Eau 70/30 vol/vol de l'exemple 1 de US7,381,436B2, sur les kératinocytes humains normaux ou KHN après 4H

**Tableau 4 :** Effet du Longoza Vert E2 et du Longoza sur les kératinocytes humains normaux

| | | | L Vert E2 0.0125% 4H | Longoza 0.0125% 4H |
|---|---|---|---|---|
| ATP synthase 6 codée dans les mitochondries | MT | + | 2,697 | - |

Le LONGOZA vert E2 selon l'exemple 2 de l'invention augmente de manière inattendue par un coefficient de +2,697 la synthèse de la sous unité synthase 6 d'ATP par les mitochondries des KHN ,

En conclusion de ce qui précède, grâce au Test ci-dessus sur KHN , il a pu être mis en évidence l'effet significativement réparateur de la dysfonction mitochondriale de l'extrait vert de *Longoza* au Butylène glycol, selon l'invention ce qui permet de lutter contre la sénescence des cellules mitochondriales et donc de lutter contre le vieillissement de la peau

Ainsi, l'invention peut être utilisée comme agent cosmétique pour la préparation de composition cosmétique à usage topique en vue de préserver les mitochondries et de lutter contre le vieillisement de la peau.

Divers exemples de formulation de composition cosmétique sont donnés ci-après à titre illustratif et qui ne sauraient donc en aucune façon limiter la portée de l'invention.

**Exemple 4 selon l'invention**

Gel cosmétique pour améliorer la fermeté du visage

| | |
|---|---|
| -extrait vert 100% butylène glycol de *Longoza* obtenu à l'exemple 1, g) | 2 % |
| - glycol | 3 % |
| polymère d'AMPS disponible dans le commerce, (dénomination commerciale Sepigel 305®) | 3 % |
| - huile de ricin hydrogénée (Cremophor CO-60®) | 2 % |
| - polyéthylène glycol | 1,5 % |
| - conservateur | 0,5 % |
| - concentré de parfum | 0,3 % |
| - filtre UV (benzophénone-4) | 1 % |

(suite)

- eau, qsp 100 %

Ce gel peut être appliqué une fois par jour pendant plusieurs semaines sur le visage afin d'aboutir à l'amélioration de la fermeté de la peau du visage, et notamment sur les zones comportant des rides, en observant ainsi à l'issue de ce traitement, une restauration de la souplesse avec un effet manifeste de rajeunissement de la peau et d'atténuation ou de disparition de ces rides.

**Exemple 5 selon l'invention**

Crème de jour anti-rides sous forme d'une émulsion

| | |
|---|---|
| - extrait vert E1 100% butylène glycol de *Longoza* obtenu à l'exemple 2 | 2 % |
| - steareth-21 (Brij 721) | 2,5 % |
| - glycéryl stéarate (Tegrin) | 1,1 % |
| - alcool stéarylique | 5 % |
| - tricaprat / caprilate glycérol | 12,5 % |
| - butylène glycol | 3 % |
| - glycérine | 2 % |
| - conservateur | 0,5 % |
| - concentré de parfum | 0,5 % |
| - filtre UV (méthoxycinnamate d'octyle) | 7,5 % |
| - eau, qsp 100 % | |

**Exemple 6 selon l'invention**

Lotion tonique anti-rides

| | |
|---|---|
| - extrait vert E2 100% butylène glycol de *Longoza* obtenu à l'exemple 2 | 2 % |
| - butylène glycol | 3 % |
| - EDTA | 0,1 % |
| - solubilisant | 1 % |
| - concentré de parfum | 0,3 % |
| - éthanol | 5 % |
| - filtre UV (benzophénone -4) | 0,13 % |
| - eau, qsp 100 % | |

**Exemple 7 selon l'invention**

Poudre de maquillage protectrice des ultraviolets pour le visage

| | |
|---|---|
| - extrait vert E3 100% butylène glycol de *Longoza* obtenu à l'exemple 2 | 0,25 % |
| - talc | 17 % |
| - mica | 20 % |
| - sericite | 20 % |
| - pigments | 8 % |
| - poudre organique (nylon) | 20 % |

(suite)

| | |
|---|---|
| - silice | 8,75 % |
| - huile minérale ou silicone | 3 % |
| - filtre UV (méthoxycinnamate d'octyle) | 3 % |

**Exemple 8 selon l'invention**

Fond de teint traitant anti-rides

| | |
|---|---|
| - extrait vert E2 100% butylène glycol de *Longoza* obtenu à l'exemple 2) | 2 % |
| - polyglycéryl-4 isostéarate et cétyl diméthicone copolyol et hexyl laurate | 5,1 % |
| - cyclopentasiloxane et cyclohexasiloxane | 5,0 % |
| - cetyl diméthicone | 1,0 % |
| - caprylic/capric triglycérides | 2,2 % |
| - octyl stéarate | 1,4 % |
| - huile minérale | 3,5 % |
| - huile de ricin hydrogénée | 1,2 % |
| - cire d'abeille | 0,8 % |
| - polyméthacrylate de méthyle | 1,1 % |
| - oxydes de fer | 0,45 % |
| - dioxydes de titane | 5,2 % |
| - NaCl | 0,6 % |
| - concentré de parfum | 0,1 % |
| - filtre UV (cinnamate d'octyle) | 3 % |
| - eau, qsp 100 % | |

**Revendications**

1. Composition cosmétique, **caractérisé en ce qu'**elle comprend, à titre d'ingrédient actif, un extrait, dit extrait vert, de graines de la plante *Aframomum angustifolium* ou *Longoza* réalisé avec un solvant polaire écologiquement compatible, et cosmétiquement acceptable, comprenant ou constitué d'un solvant polaire de polyol, en particulier un glycol, dans un excipient cosmétiquement acceptable.

2. Composition selon la revendication 1, **caractérisée en ce que** l'extrait des graines de la plante est obtenu par un procédé d'extraction comprenant au moins une étape d'extraction des graines broyées avec le solvant polaire de polyol, en particulier un glycol.

3. Composition selon la revendication 1 ou 2, **caractérisée** en ce l'extrait des graines de la plante est dissout à nouveau dans un solvant compatible avec l'environnement et cosmétiquement acceptable, en particulier comprenant un polyol, plus particulièrement un glycol, ou un alcool ou un mélange hydro-alcoolique.

4. Composition selon la revendication 1 ou 2 ou 3, **caractérisée en ce que** le polyol présente la formule chimique générale HO-R-OH, dans laquelle R peut représenter un radical hydrocarboné -(CH2)n avec n ayant de 2 à 16 atomes de carbone, en particulier de 3 à 8 atomes de carbone.

5. Composition selon la revendication 4, **caractérisée en ce que** le polyol , comprend un glycol, par exemple choisi parmi le propylène glycol, le butylène glycol ou 1, 3-butylène glycol, le pentylène glycol, et leur(s) mélanges.

6. Composition selon l'une des revendications 1 à 5, **caractérisée en ce que** l'extrait précité de graines de *Longoza* , , est présent à une concentration, exprimée en extrait sec, comprise entre 0,001 % et 5 %, de préférence entre 0,01 % et 1 %, en poids par rapport au poids total de la composition.

**7.** Utilisation d'un extrait, dit extrait vert, de graines de la plante *Aframomum angustifolium* ou *Longoza* réalisé avec un solvant polaire écologiquement compatible, et cosmétiquement acceptable, comme agent cosmétique, pour la fabrication d'une composition cosmétique, ledit extrait étant tel que défini à l'une quelconque des revendications 1 à 6.

**8.** Utilisation selon la revendication 7, **caractérisée en ce que** l'extrait de *Longoza* est utilisé pour une activité anti-vieillissement, en particulier par une action de stimulation de la sous-unité synthase 6 de l'ATP.

**9.** Procédé de soin cosmétique, **caractérisé en ce qu'**il comprend l'application topique sur la peau d'une personne en ayant besoin, d'une quantité cosmétiquement efficace d'un extrait, dit extrait vert, de graines de la plante *Aframomum angustifolium* ou *Longoza* tel que défini à l'une quelconque des revendications 1 à 6.

**10.** Procédé selon la revendication 9, **caractérisé en ce qu'**on réalise l'application de l'extrait de graines de la plante *Aframomum angustifolium* ou *Longoza* précité, sur les zones de la peau ayant besoin d'un soin anti-vieillissement, en particulier une activité anti-rides, pour diminuer, éliminer, ou ralentir l'apparition de rides, ou pour lutter contre les effets du vieillissement résultant d'un dysfonctionnement des mitochondries, ou par une action préservant ou restaurant la structure de la peau, un effet de protection, de réparation ou de restauration de l'élasticité et/ou de la fermeté de la peau, notamment par une action de stimulation de la sous-unité synthase 6 de l'ATP.

**Patentansprüche**

**1.** Kosmetische Zusammensetzung, **dadurch gekennzeichnet, dass** sie als Wirkstoff einen Extrakt, einen so genannten grünen Extrakt, von Samen der Pflanze *Aframomum angustifolium* oder *Longoza,* der mit einem ökologisch verträglichen und kosmetisch annehmbaren polaren Lösungsmittel, das ein polares Polyol-Lösungsmittel, insbesondere ein Glykol, umfasst oder daraus besteht, hergestellt wird, in einem kosmetisch annehmbaren Hilfsstoff umfasst.

**2.** Zusammensetzung nach Anspruch 1, **dadurch gekennzeichnet, dass** der Extrakt von Samen der Pflanze durch ein Extraktionsverfahren erhalten wird, das mindestens einen Schritt der Extraktion von zerstoßenen Samen mit dem polaren Polyol-Lösungsmittel, insbesondere einem Glykol, umfasst.

**3.** Zusammensetzung nach Anspruch 1 oder 2, **dadurch gekennzeichnet, dass** der Extrakt von Samen der Pflanze erneut in einem umweltverträglichen und kosmetisch annehmbaren Lösungsmittel, das insbesondere ein Polyol, ganz besonders ein Glykol, oder einen Alkohol oder ein wässrig-alkoholisches Gemisch umfasst, gelöst wird.

**4.** Zusammensetzung nach Anspruch 1 oder 2 oder 3, **dadurch gekennzeichnet, dass** das Polyol die allgemeine chemische Formel HO-R-OH aufweist, in der R für einen Kohlenwasserstoffrest $-(CH_2)_n$ stehen kann, wobei n 2 bis 16 Kohlenstoffatome, insbesondere 3 bis 8 Kohlenstoffatome aufweist.

**5.** Zusammensetzung nach Anspruch 4, **dadurch gekennzeichnet, dass** das Polyol ein Glykol umfasst, das beispielweise aus Propylenglykol, Butylenglykol oder 1,3-Butylenglykol, Pentylenglykol und seinen (ihren) Gemischen ausgewählt ist.

**6.** Zusammensetzung nach einem der Ansprüche 1 bis 5, **dadurch gekennzeichnet, dass** der oben genannte Extrakt von Samen von *Longoza* in einer Konzentration, ausgedrückt als Trockenextrakt, vorliegt, die zwischen 0,001 Gew.-% und 5 Gew.-%, vorzugsweise zwischen 0,01 Gew.-% und 1 Gew.-%, bezogen auf das Gesamtgewicht der Zusammensetzung, liegt.

**7.** Verwendung eines Extrakts, eines so genannten grünen Extrakts, von Samen der Pflanze *Aframomum angustifolium* oder *Longoza,* der mit einem ökologisch verträglichen und kosmetisch annehmbaren polaren Lösungsmittel hergestellt wird, als Kosmetikum zur Herstellung einer kosmetischen Zusammensetzung, wobei der Extrakt wie in einem der Ansprüche 1 bis 6 definiert ist.

**8.** Verwendung nach Anspruch 7, **dadurch gekennzeichnet, dass** der Extrakt von *Longoza* für eine Anti-Aging-Aktivität, insbesondere eine Wirkung zur Stimulierung der ATP-Synthase-Untereinheit 6 verwendet wird.

**9.** Verfahren zur kosmetischen Pflege, **dadurch gekennzeichnet, dass** es die topische Anwendung einer kosmetisch wirksamen Menge eines Extrakts, eines so genannten grünen Extrakts, von Samen der Pflanze *Aframomum an-*

*gustifolium* oder *Longoza,* wie in einem der Ansprüche 1 bis 6 definiert, auf die Haut einer Person, die dieser bedarf, umfasst.

10. Verfahren nach Anspruch 9, **dadurch gekennzeichnet, dass** man die Anwendung des oben genannten Extrakts von Samen der Pflanze *Aframomum angustifolium* oder *Longoza* auf die Bereiche der Haut, die einer Anti-Aging-Pflege, insbesondere einer Anti-Falten-Aktivität bedürfen, durchführt, um das Auftreten von Falten zu verringern, auszumerzen oder zu verzögern oder um die Alterungseffekte, die aus einer mitochondrialen Dysfunktion resultieren, zu bekämpfen, oder um einen Effekt zum Schutz, zur Reparatur oder zur Wiederherstellung der Elastizität und/oder der Festigkeit der Haut durch eine Wirkung, die die Struktur der Haut bewahrt oder wiederherstellt, insbesondere durch eine Wirkung zur Stimulierung der ATP-Synthase-Untereinheit 6, zu erreichen.

**Claims**

1. A cosmetic composition, **characterized in that** it comprises, as active ingredient, an extract, called green extract, of seeds of the *Aframomum angustifolium* or longoza plant made with an ecologically friendly and cosmetically acceptable polar solvent, comprising or consisting of a polar solvent of polyol, in particular a glycol, in a cosmetically acceptable excipient.

2. The composition as claimed in claim 1, **characterized in that** the extract from the seeds of the plant is obtained by an extraction process comprising at least one step of extracting the ground seeds with the polar solvent of polyol, in particular a glycol.

3. The composition as claimed in claim 1 or 2, **characterized in that** the extract from the seeds of the plant is dissolved again in an environmentally friendly and cosmetically acceptable solvent, in particular comprising a polyol, more particularly a glycol, or an alcohol or a hydroalcoholic mixture.

4. The composition as claimed in claim 1 or 2 or 3, **characterized in that** the polyol has the general chemical formula HO-R-OH, wherein R can represent a hydrocarbon radical $-(CH_2)_n$ with n having 2 to 16 carbon atoms, in particular 3 to 8 carbon atoms.

5. The composition as claimed in claim 4, **characterized in that** the polyol comprises a glycol, for example selected from propylene glycol, butylene glycol or 1,3-butylene glycol, pentylene glycol, and mixtures thereof.

6. The composition as claimed in one of claims 1 to 5, **characterized in that** said extract of longoza seeds is present at a concentration, expressed as dry extract, of between 0.001% and 5%, preferably between 0.01% and 1%, by weight based on the total weight of the composition.

7. Use of an extract, called green extract, of seeds of the *Aframomum angustifolium* or longoza plant made with an ecologically friendly and cosmetically acceptable polar solvent, as a cosmetic agent, for the manufacture of a cosmetic composition, said extract being as defined in any one of claims 1 to 6.

8. Use as claimed in claim 7, **characterized in that** the longoza extract is used for an anti-aging effect, in particular by a stimulating action of the ATP synthase subunit 6.

9. A cosmetic care process, **characterized in that** it comprises the topical application to the skin of a person in need thereof of a cosmetically effective amount of an extract, called green extract, of seeds of the *Aframomum angustifolium* or longoza plant as defined in any one of claims 1 to 6.

10. The process as claimed in claim 9, **characterized in that** the application of the seed extract of the *Aframomum angustifolium* or longoza plant is carried out on the areas of the skin in need of an anti-aging care, in particular an anti-wrinkle effect, to reduce, eliminate or slow the appearance of wrinkles, or to combat the effects of aging resulting from mitochondrial dysfunction,
or by an action preserving or restoring the structure of the skin, a protective, repairing or restoring effect on the elasticity and/or firmness of the skin, in particular by a stimulating action of the ATP synthase subunit 6.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 7381436 B2, ANDRE
- US 20100055217 A
- US 7381436 B

**Littérature non-brevet citée dans la description**

- **E.M. GAYDOU et al.** *Revue française des Corps Gras,* Janvier 1983, (1), 21-25
- **WILEY et al.** *Cell Metab,* 09 Février 2016, vol. 23 (2), 303-314